(19) **Europäisches Patentamt / European Patent Office / Office européen des brevets**

(11) **EP 4 556 570 A1**

(12) **EUROPEAN PATENT APPLICATION**

(43) Date of publication:
**21.05.2025   Bulletin 2025/21**

(21) Application number: **23210958.7**

(22) Date of filing: **20.11.2023**

(51) International Patent Classification (IPC):
**C12P 19/04** $^{(2006.01)}$

(52) Cooperative Patent Classification (CPC):
**C12P 19/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **ETH Zurich**
**8092 Zurich (CH)**

(72) Inventors:
- **STUDART, André**
  **8044 Zürich (CH)**
- **DEMELLO, Andrew**
  **8044 Zürich (CH)**

- **STAVRAKIS, Stavros**
  **8044 Zürich (CH)**
- **LAURENT, Julie**
  **8050 Zürich (CH)**
- **KAN, Anton**
  **8050 Zürich (CH)**
- **JAIN, Ankit**
  **8057 Zürich (CH)**
- **STEINACHER, Mathias**
  **3008 Bern (CH)**
- **ENRRIQUEZ, Nadia**
  **8050 Zürich (CH)**

Remarks:
The complete document including Reference Table(s) and the Sequence Listing(s) can be downloaded from the EPO website

(54) **IMPROVED PRODUCTION OF BACTERIAL CELLULOSE**

(57)   The invention concerns a genetically modified cellulose producing bacterial strain comprising a reduction in expression of native gene encoding a ClpS polypeptide, or a deletion of the native gene encoding a native ClpS polypeptide, or comprising expressing or overexpressing a gene encoding a ClpA polypeptide containing a mutation or a deletion in the ClpS-binding region of said polypeptide, and/or expressing or overexpressing a gene encoding a ClpS polypeptide containing a mutation or deletion in the ClpA-binding region of said polypeptide, causing an alteration of binding affinity between the ClpS polypeptide and the ClpA polypeptide.

Fig. 4c

EP 4 556 570 A1

## Description

## Technical domain

The present disclosure concerns a genetically modified microorganism with improved cellulose production. The present disclosure also concerns a method of producing cellulose.

## Related art

Compared to plant-based cellulose products, which often contain a significant amount of lignin, pectin, hemicellulose, and other biogenic products, bacterial cellulose (BC), which also referred to as microbial cellulose, can be produced sustainably at a high degree of purity in a bacterial fermentation process. Further remarkable properties of BC include its high degree of biocompatibility, its high tensile strength, its high modulus of elasticity as well as its high crystallinity and high water holding capacity. Its distinct advantages render it highly attractive for diverse industrial applications, such as in medical products, food products, personal care products, textile products and in composite resins.

Several Gram-negative bacterial species have been reported to synthesize cellulose, such as *Gluconacetobacterxylinus,* also known as *Acetobacterxylinum,* or certain species of the bacterial genera *Agrobacterium* spp., for example *Agrobacterium tumefaciens, Acetobacter* spp., *Azotobacter, Rhizobium* spp., *Sarcina, Alcaligenes, Pseudomonas, and Komagataeibacter,* for example *Komagataeibacter sucrofermentans.*

In addition, biosynthesis of BC in *Escherichia coli,* which is not a natural cellulose producer, by recombinant expression of the BC synthase operon *bcsABCD,* has also been reported (Buldom G et al, Bioprocess and Biosystems Eng (2018) 41:265-279).

Despite the commercial interest in producing cellulose by bacterial fermentation, industrial production of BC is still relatively costly, due to expensive medium components and the generally low production yields. Moreover, the production of BC usually occurs at the air-water interface of cultures, thus limiting the quantity of cellulose which can be produced per culture volume.

It is therefore highly desirable to render the production of bacterial cellulose more efficient.

## Short disclosure of the invention

It is an object of the present invention to increase the yield in bacterial cellulose production.

It is yet another aim to provide an improved production method for bacterial cellulose with increased cellulose yield.

According to the invention, one or more of these aims are attained by the object of the attached claims, and especially by a genetically modified cellulose-producing bacterial strain having a reduction in expression of native gene encoding a ClpS polypeptide, or a deletion of the native gene encoding a native ClpS polypeptide.

Alternatively, the genetically modified cellulose-producing bacterial strain of this invention comprises a mutation or a deletion in the ClpS binding region of an expressed or overexpressed ClpA polypeptide, and/or a mutation or a deletion in the ClpA binding domain of an expressed or overexpressed ClpS polypeptide, said mutations or deletions causing an alteration in binding affinity between the ClpS polypeptide and the ClpA polypeptide.

In one embodiment the alteration is an abolishment of binding affinity between the ClpS polypeptide and the ClpA polypeptide. In an alternative embodiment the alteration is a reduction in binding affinity between the ClpS polypeptide and the ClpA polypeptide.

In one embodiment, the genetically modified bacterial strain contains a mutation or a deletion in a nucleotide sequence of a homolog of the *E. coli clpA* gene sequence, said nucleotide sequence encoding the conserved arginine (R) residue in the ClpS-binding region of the ClpA polypeptide, said R residue corresponding to the R86 residue in the *E. coli* ClpA polypeptide.

The genetically modified bacterial strain may comprise a mutation or a deletion in a sequence of at least twelve nucleotides of a homolog of the *E. coli clpA* gene sequence, said twelve nucleotides encoding four amino acids of the ClpS-binding region of the ClpA polypeptide, said four amino acids corresponding to residues 85 to 88 in the *E. coli* ClpA polypeptide.

A deletion of the highly conserved R86 residue in a homolog of the *E. coli* ClpA polypeptide has previously been shown to abolish ClpS binding to ClpA. It is therefore to be expected, that a deletion of this conserved residue on its own or together with its adjacent residues results at least in a reduced binding affinity between ClpS and ClpA homologs in other bacterial species.

In one embodiment of this invention binding of the ClpS polypeptide to the N-terminal region of the ClpA polypeptide is abolished.

In one embodiment, the genetically modified bacterial strain contains a mutation or a deletion in a nucleotide sequence of a homolog of the *E. coli clpA* gene sequence, said nucleotide sequence encoding one of the two conserved glutamic acid (E) residues in the ClpS-binding region of the ClpA polypeptide, said conserved E residues corresponding to the E23 residue and to the E28 residue in the homologous *Escherichia coli* ClpA polypeptide. The modified bacterial strain may comprise mutations or deletions in both of said nucleotide sequences.

In one embodiment, the genetically modified bacterial strain contains a mutation or a deletion in a nucleotide sequence of a homolog of the *E. coli clpA* gene sequence, said nucleotide sequence encoding the conserved threonine (T) residue in the ClpS-binding region of the ClpA polypeptide, said T residue corresponding to the T81 residue in the *E. coli* ClpA polypeptide.

In addition or alternatively, the genetically modified bacterial strain may comprise a mutation or a deletion of one or more of the conserved residues in the gene sequence encoding other conserved residues in the ClpA-binding region of the ClpS polypeptide.

In one embodiment, the genetically modified bacterial strain contains a mutation or a deletion in a nucleotide sequence of a homolog of the *E. coli clpS* gene sequence, said nucleotide sequence encoding one of the two conserved glutamic acid (E) residues in the ClpA-binding region of the ClpS polypeptide, said conserved E residues corresponding to the E79 residue and to the E82 residue in the homologous *Escherichia coli* ClpS polypeptide. The modified bacterial strain may comprise mutations or deletions in both of said nucleotide sequences.

In one embodiment, the genetically modified bacterial strain contains a mutation or a deletion in a nucleotide sequence of a homolog of the *E. coli clpS* gene sequence, said nucleotide sequence encoding the conserved lysine (K) residue in the ClpA-binding region of the ClpS polypeptide, said K residue corresponding to the K84 residue in the *E. coli* ClpS polypeptide.

In one embodiment, the binding affinity of the ClpS polypeptide to the ClpA polypeptide is reduced by at least 20%, at least 30%, at least 40%, at least 50%, at least 60%, at least 70%, at least 80%, or at least 90%.

The ClpA polypeptide is part of the ClpAPS complex, which is responsible for the hydrolysis of misfolded and degraded proteins inside cells. ClpS is an adapter which modifies ClpA substrate specificity. It was surprisingly found as part of this invention, that mutations or deletions which alter, in particular mutations or deletions which reduce or abolish binding between ClpS and ClpA improve the yield of cellulose in a cellulose-producing bacterial strain.

In one embodiment of this invention, the protease activity of the ClpAP protein complex is reduced. The protease activity may be reduced by at least 20%, at least 30%, at least 40%, at least 50%, at least 60%, at least 70%, at least 80%, or at least 90%.

In one embodiment the genetically modified bacterial strain is derived from a naturally cellulose-producing parental strain. The genetically modified bacterial strain may naturally comprise the *bcsA, bcsB, bcsC* and *bcsD* genes or homologs thereof encoding subunits of the cellulose synthase complex.

The parental strain may be a naturally cellulose-producing strain selected from a group consisting of *Komagataeibacter* sp, for example *K. sucrofermentans, Gluconacetobacter* sp, for example *G. xylinus, Agrobacterium* spp., for example *A. tumefaciens, Acetobacter* spp., *Azotobacter, Rhizobium* spp., *Sarcina, Alcaligenes,* and *Pseudomonas.*

The genetically modified bacterial strain may be the strain designated K. *sucrofermentans* JML 2321 having CCOS accession number CCOS 2107, or a *K. sucrofermentans* strain having a growth profile and a cellulose production profile of CCOS deposit accession number CCOS 2107.

It is however also posible, that the parental wild-type (wt) strain is not a naturally cellulose producing strain. Such wt strain may be transformed with genes encoding the cellulose synthase complex, such as to generate a recombinant bacterial strain which synthesises cellulose. Such genes may for example be a *bcsA,* a *bcsB,* a *bcsC* and a *bcsD* gene or homologs thereof derived from a naturally cellulose-producing strain, causing the recombinant strain to express the BcsA, BcsB, BcsC and BcsD subunits or homologs thereof of the cellulose synthase complex. In addition, said wt strain may be transformed with and heterologously expressing a *cmcax* and a *ccpAx* gene.

It has been previously described, that trait of cellulose production can be introduced in bacterial strains by genetic engineering. This invention concerns additional mutations which improve the yield in cellulose production. It may be applied to naturally as well as recombinant cellulose producing bacterial strains.

In one embodiment the genetically modified bacterial strain of this invention produces at least 15%, or at least 20%, 30%, 40%, 50%, 60%, 70%, 80%, 90%, 100% more cellulose that its parental strain. The parental strain may be a naturally cellulose producing wt strain, or a recombinant strain which produces cellulose.

The invention also concerns a method for producing cellulose in a bacterial fermentation process using a genetically modified strain of this invention.

In one embodiment, the genetically engineered bacterial strain is grown in a static liquid culture. The produced bacterial cellulose is generally produced at the surface of the liquid culture, i.e. at the liquid/air interface. The produced cellulose forms a film or a pellicle at this interface.

In one embodiment, the genetically engineered bacterial strain is grown in an agitated liquid culture. The culture may be stirred or shaken during its incubation. Cellulose produced in the agitated culture forms cellulose pellets or small granules.

The temperature and growth medium for the fermentation are chosen according to the species of the cultivated strain. Optionally, oxygen and additional nutrients may be provided to the liquid culture during fermentation to further improve the efficiency of the production process.

The genetically modified strain of this invention may be used to create cellulose-based macroscopic objects. Such cellulose-based macroscopic objects may be generated using state-of-the-art manufacturing technologies, such as casting, molding and 3D printing. By combining the bottom-up cellulose-producing capability of the modified bacterial strain of this invention with the top-down shaping freedom of conventional manufacturing, living materials with unique multiscale architectures can be created.

So that the invention may be more readily understood, certain terms are defined hereinunder.

The term "gene" refers to a polynucleotide that codes for a particular sequence of amino acids, which comprise all or part of one or more proteins or enzymes, and may include regulatory (non- transcribed) DNA sequences, such as promoter sequences, which determine for example the conditions under which the gene is expressed. The transcribed region of the gene may include untranslated regions, including introns, 5'-untranslated region (UTR), and 3'-UTR, as well as the coding sequence.

The term "expression" with respect to a gene sequence refers to transcription of the gene and, as appropriate, translation of the resulting mRNA transcript to a protein. Thus, as will be clear from the context, expression of a protein results from transcription and translation of the open reading frame sequence.

The term "bacterial strain" refers to a genetic variant, a subtype or a culture within a biological species. A bacterial strain differs from other bacteria of the same species by one or more identifiable differences.

A "genetically modified" bacterial strain or cell is a bacterial strain or cell whose genome has been engineered or evolved in the laboratory in order to favour the expression of desired physiological traits. The terms "evolved" or "mutant" strains or cells as used herein refer to a genetically modified strains or cells.

The term "recombinant bacterial strain" and "recombinant bacterial cell" are used interchangeably herein and refer to a bacterial strain or cells of a bacterial strain that have been genetically modified to express or over-express endogenous polynucleotides, or to express non- endogenous sequences, such as those included in a vector, or which have a reduction in expression of an endogenous gene. It is understood that the terms "recombinant bacterial strain", "recombinant strain" and "recombinant cell" refer not only to the particular recombinant microorganism but to the progeny or potential progeny of such a microorganism.

A "parental bacterial strain" or "parental strain" refers to a strain, respectively a cell of a strain, used to generate a genetically modified bacterial strain. The term "parental" refers to a cell that occurs in nature, i.e. a "wild-type" (wt) strain that has not been genetically modified. The term "parental" also describes a strain that has been genetically modified. For example, a wt strain can be genetically modified to express or overexpress a first target polypeptide or polypeptide complex, such as the BcsABCD complex. This bacterial stain can act as a parental bacterial strain in the generation of a bacterial strain modified to express or overexpress a second target polypeptide etc. Accordingly, a parental bacterial strain functions as a reference strain for successive genetic modification events. Each modification event can be accomplished by introducing a nucleic acid molecule into a cell of the reference strain. The introduction facilitates the expression or over-expression of a target polypeptide. It is understood that the term "facilitates" encompasses the activation of endogenous polynucleotides encoding a target polypeptide through genetic modification of e.g., a promoter sequence in a parental microorganism. It is further understood that the term "facilitates" encompasses the introduction of exogenous polynucleotides encoding a target polypeptide into a parental bacterial strain.

The term "native strain" as used herein means the parental strain which was not genetically modified, in the present study the parental strain K. *sucrofermentans* strain ATCC 700178.

The term "operon" refers to two or more genes which are transcribed as a single transcriptional unit from a common promoter. In some embodiments, the genes comprising the operon are contiguous genes. It is understood that transcription of an entire operon can be modified (i.e., increased, decreased, or eliminated) by modifying the common promoter.

A "protein" or "polypeptide", which terms are used interchangeably herein, comprises one or more chains of chemical building blocks called amino acids that are linked together by chemical bonds called peptide bonds.

The disclosure also provides a deposited bacterial strain. The deposited bacterial strain is exemplary only and, based upon the disclosure, one of ordinary skill in the art can modify additional parental bacterial organisms of different species or genotypes to arrive at a bacterial strain of the disclosure, which exhibits improved bacterial cellulose synthesis.

The term "homolog" used with respect to an original polypeptide or gene of a first family or species refers to distinct polypeptides or genes of a second family or species which are determined by functional, structural or genomic analyses to be a polypeptide or gene of the second family or species which corresponds to the original polypeptide or gene of the first family or species. Most often, homologs will have functional, structural or genomic similarities. Techniques are known by which homologs of a polypeptide or gene can readily be cloned using genetic probes and PCR. Identity of cloned sequences as homolog can be confirmed using functional assays and/or by genomic mapping of the genes.

The singular "a", "an" and "the" include the plural equivalents unless the context distinctly indicates otherwise. Likewise, "or" is intended to include "and" unless the context distinctly indicates otherwise.

The terms "comprises" or "contains" means "includes" in a non-limiting sense.

The terms "for example" and "such as" are used herein to indicate a non-limiting example.

## Short description of the drawings

Exemplar embodiments of the invention are disclosed in the description and illustrated by the drawings in which:

**Figure 1a** is a diagram showing the survival rates of cell suspensions exposed to different UV-C doses compared to a non-exposed sample; the culture exposed to 10 mJ/cm$^2$ (indicated by an arrow) was selected for the directed evolution process;

**Figure 1b** is a diagram depicting the impact of a cell recovery step in the erected evolution process on the cell concentration after mutagenesis; error bars represent the standard deviation;

**Figure 2** illustrates the change in quantity of fluorescently-labelled cellulose over time, presenting the cellulose-correlated fluorescence in individual droplets as determined by confocal microscopy for about 1'200 sample droplets, with error bars corresponding to standard error of the mean;

**Figures 3a to 3f** show single-cell laden droplets at different incubation times, respectively quantified cellulose production in the droplets at different incubation times, with

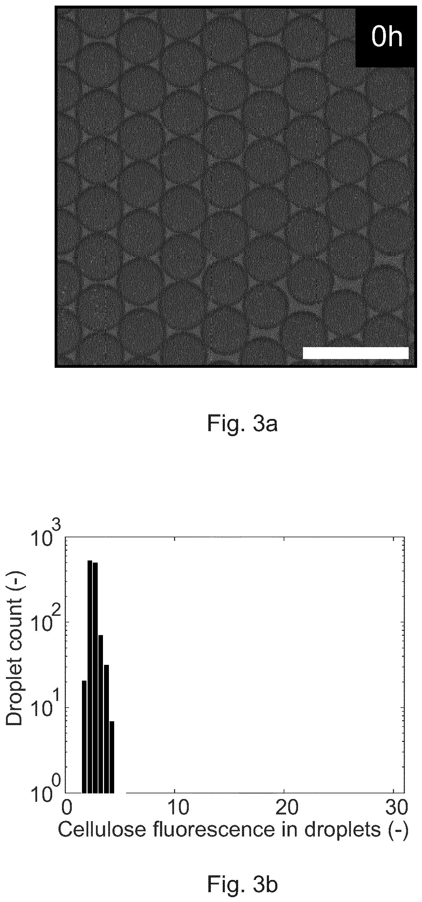

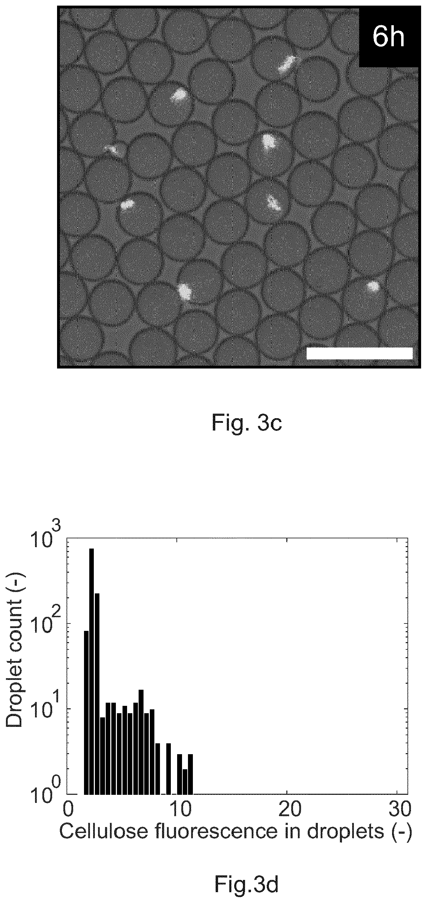

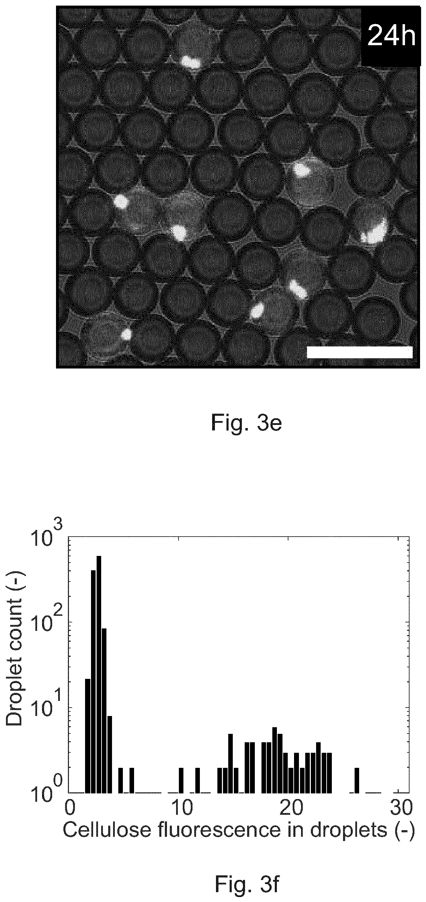

**Figures 3a, 3c and 3e** showing representative confocal microscopy images taken after 0h, 6h and 24h incubation time, as indicated, with fluorescently labelled cellulose in cyan (excitation: 405 nm; emission: 432-460 nm) shown in fair grey in the dark grey droplets, the scale bar corresponding to 100 $\mu$m;

**Figures 3b, 3d, and 3f** showing the corresponding histograms of cellulose fluorescence per droplet, quantified by image analysis (n ~1200 droplets) after 0h, 6h and 24h incubation time, as indicated;

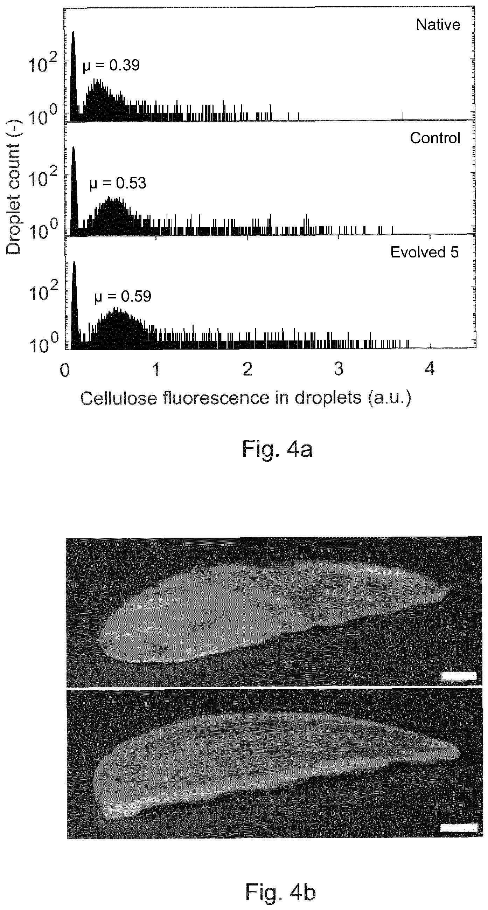

**Figure 4a** shows a histograms of cellulose fluorescence signal in droplets containing either the native *K. sucrofermentans* strain ATCC 700178, the control sample which was not irradiated, or the evolved *K. sucrofermentans* strain Ev5 ($\lambda$ ~0.1 CFU/droplet, 10'000 droplet events);

**Figure 4b** shows images of washed and freeze-dried bacterial cellulose pellicles having a diameter of 9.65 cm, produced in 100ml cultures of the native parental *K. sucrofermentans* strain and by the evolved *K. sucrofermentans* Ev5 strain, with the scale bar corresponding to 1 cm;

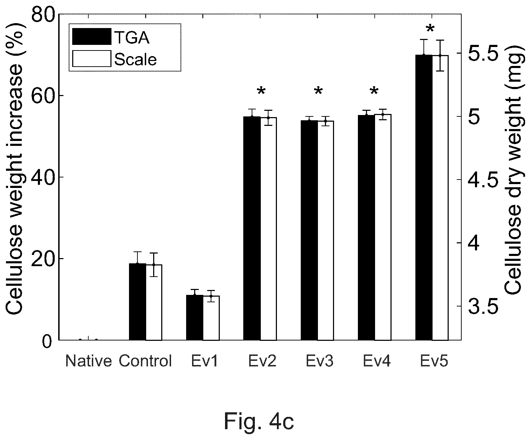

**Figure 4c** weight measurements of washed and air-dried bacterial cellulose pellicles grown for 12 days from single colonies having a diameter of 3 cm, grown in 5 ml cultures at 28°C under static conditions; weight was measured with both a scale and via thermogravimetric analysis (TGA) for each sample; error bars correspond to the error propagation; evolved strains (Ev2, Ev3, Ev4, and Ev5) showed significantly increased cellulose production (54-70%) compared to the native strain (*p < 0.05, n = 3);

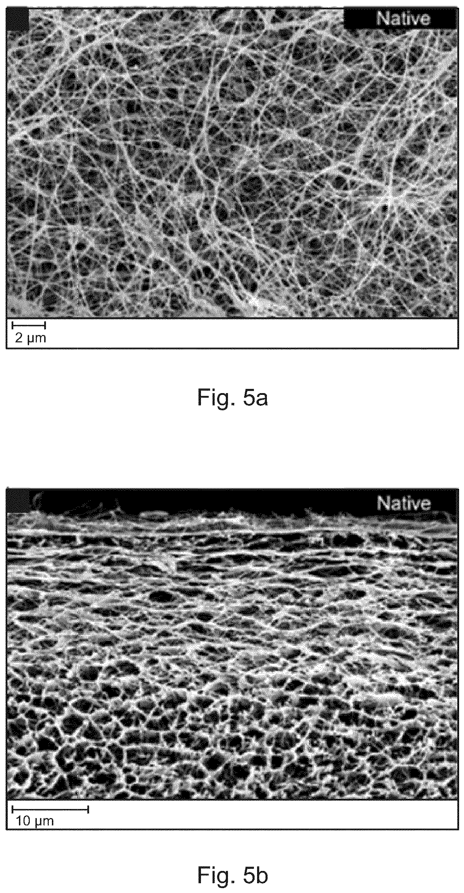

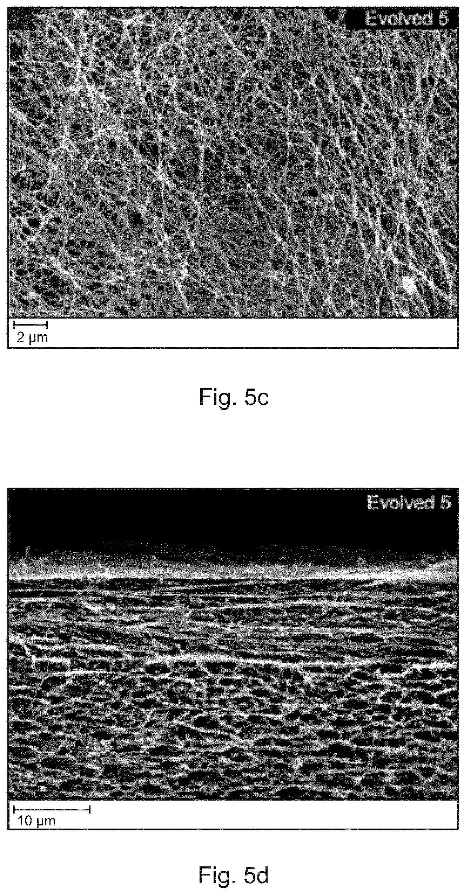

**Figure 5a to d** show scanning electron microscopy images of freeze-dried bacterial cellulose pellicles produced by the native *K. sucrofermentans* strain JCM 9730 (ATCC 700178) **(Figures 5a and 5b)** and *K. sucrofermentans* strain JML 2321 (Ev5) **(Figures 5c and 5d)** after 12 days of incubation at 28°C under static conditions, with **Figures 5a and 5c** representing the bottom fibers of the pellicle, and **Figures 5b and 5d** showing cross sections of the pellicle;

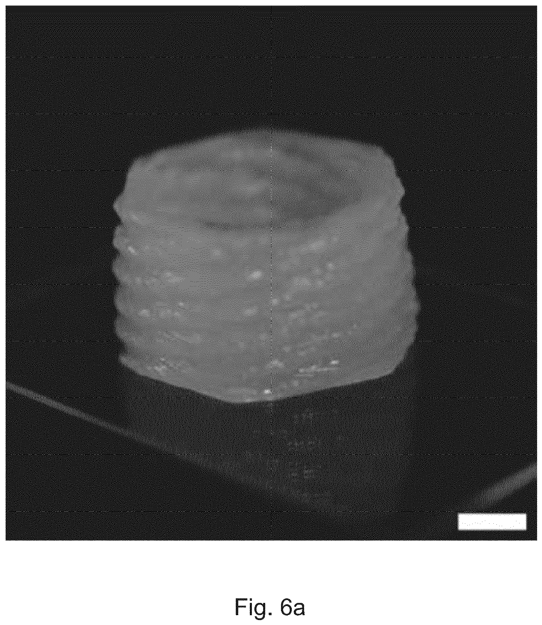

**Figure 6a** is an image of a centimeter-scale engineered living object obtained by 3D printing the bacteria-laden ink, the scale bar corresponding to 1 cm;

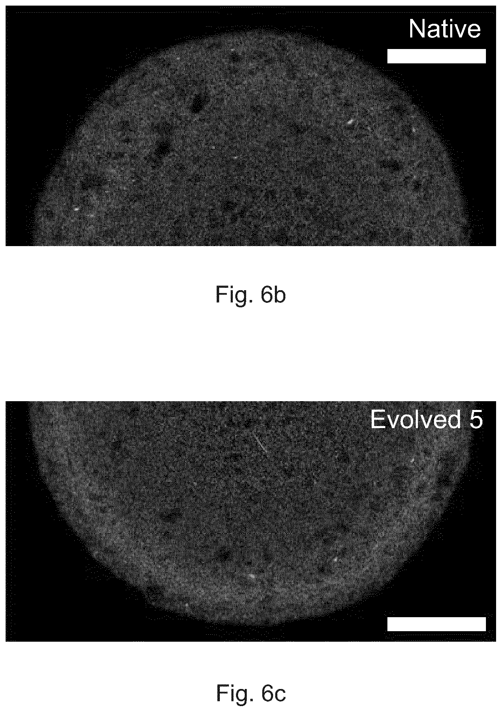

**Figure 6b** is a representative stitched confocal image of fluorescently labeled bacterial cellulose (excitation: 405 nm; emission: 432-460) in the 3D-printed disks containing the native parental *K. sucrofermentans* strain after one day of incubation; the scale bar corresponding to 3 mm;

**Figure 6c** is a representative stitched confocal image of fluorescently labeled bacterial cellulose (excitation: 405 nm; emission: 432-460) in the 3D-printed disks containing the evolved *K. sucrofermentans strain* JML 2321 (Ev5) after one day of incubation; the scale bar corresponding to 3 mm;

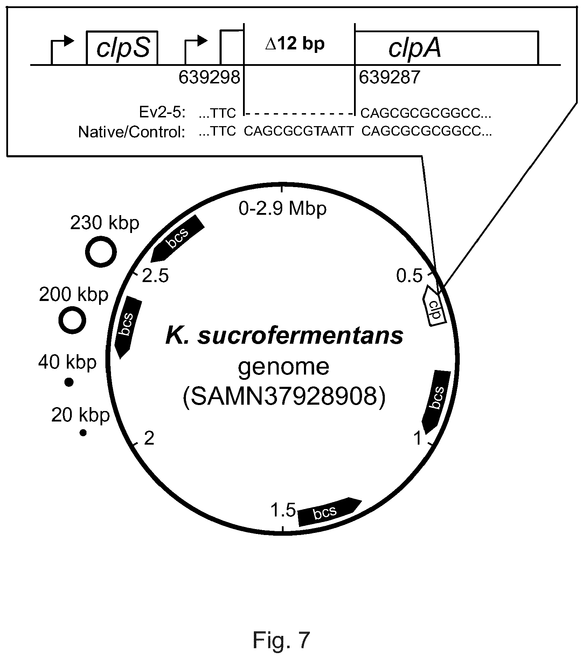

**Figure 7** is a schematic genome representation of the native *K. sucrofermentans* strain ATCC 700178 and the evolved *K. sucrofermentans* strain JML 2321, which contains a 12-bp deletion in *clpA* gene encoding the ClpA polypeptide of the ClpAPS protease complex; genes are not to scale;

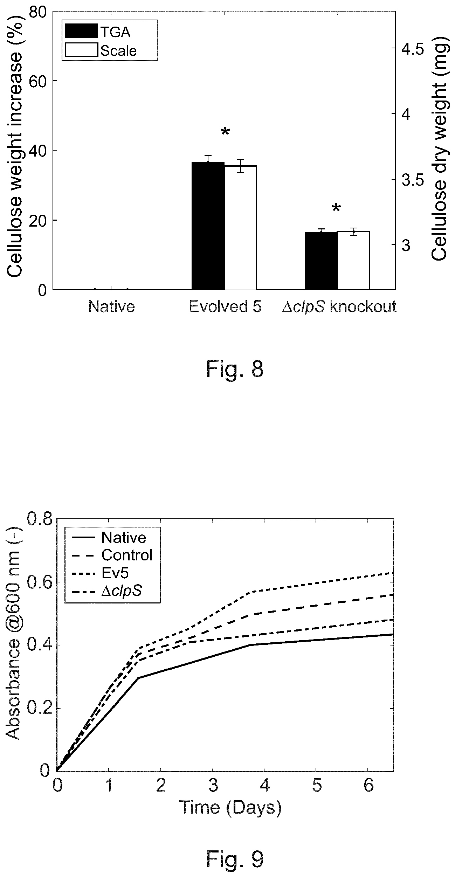

**Figure 8** shows TGA and scale weight measurements of washed and air-dried bacterial cellulose pellicles (diameter of 3 cm, 5 ml growth media, 28°C, static culture conditions) produced by the native *K. sucrofermentans* strain ATCC 700178, the evolved *K. sucrofermentans* strain Ev5, and the *K. sucrofermentans* Δ*clpS* knockout after 12 days of incubation from single colonies, with error bars correspond to the error propagation; both the evolved Ev5 strain and the Δ*clpS* knockout strains showed increased cellulose production, which were 37% and 17% higher compared to the native strain; statistical analysis indicates a significantly different amount of cellulose produced by the different strains (*p < 0.05, n = 5).

**Figure 9** shows bacterial growth curves of the native *K. sucrofermentans* strain ATCC 700178, the evolved *K. sucrofermentans* strain Ev5, the control strain, which was not irradiated with UV-C light, and the *K. sucrofermentans* ΔclpS knockout in the presence of 2 vol% cellulase in shaking conditions (200 rpm, 28°C, 80% relative humidity), (n = 3).

**Examples of embodiments of the present invention**

*Komagataeibacter sucrofermentans* was used as a test strain to identify suitable genetic modifications which result in increased cellulose production. *K. sucrofermentans* is a Gram-negative aerobic bacterium that produces long cellulose nanofibers found in food products, wound dressing materials and high-end acoustic membranes.

*K. sucrofermentans* metabolizes sugars to UDP-glucose, which is then catalyzed by the transmembrane cellulose synthase complex into β(1-4) glucan chains that self-assemble into cellulose fibers while exported through the cell wall. The core cellulose synthase machinery in *K. sucrofermentans* is encoded by multiple copies of the *bcsA, bcsB,* the *bcsC* homolog *ascC,* and the *bcsD* homolog *ascD,* which are present in several operons throughout the genome [SEQ ID NO 1, 2, 3 and 4], as depicted in Figure 7. Since the function of some of those genes are not yet fully understood and that other genes are expected to be involved in the regulation of the cellulose formation process, a whole-genome directed evolution approach was chosen for the generation of mutant strains with the desired traits, i.e. an increase in cellulose production.

The directed evolution process described herein aimed to boost bacterial cellulose production within a 24 hour fermentation time frame. The aim was to generate strains comprising one or more genetic mutations resulting in increased cellulose production of the mutated strain compared to its native parental strain. To enhance the efficacy of the mutation and selection process, the direct evolution process was combined with a microfluidic approach. The mutagenic approach is further detailed in the methods section below.

The approach taken was to enhance the bacterial production of cellulose by exploring the entire genome of the microorganism rather than introducing mutations only in the genes encoding a specific target protein. To this end, a random mutagenesis approach using UV light was chosen and a library of mutants was created. This approach allowed to tap into the broad diversity of possible genetic mutants without prior assumptions about the genes controlling cellulose bio-synthesis. From this initial mutant library, mutants with the desired phenotype were selected by performing direct evolution cycles in a droplet microfluidic platform as described in the material and methods section. Encapsulating a single bacterium in individual droplets provides the advantage that a direct link between genotype of the single bacterium and its phenotype is directly established.

The microfluidic platform used herein consisted of three parts: a droplet generator for cell encapsulation, an incubation container for cell growth, and a high-speed droplet sorter.

To enable directed evolution of individual cells, one microorganism per droplet was encapsulated. This was achieved by adjusting the initial cell concentration in the culture medium accordingly. After encapsulation, cell-laden droplets were incubated in glass containers for 1-3 days at optimum growth conditions in the presence of a cellulose-binding fluorescent dye.

Droplets containing mutants that overproduce cellulose were dielectrophoretically separated from the remainder of the population by applying an electrical pulse. The electrical pulse was triggered when the measured fluorescence of the droplet exceeded a defined threshold level.

Sorted droplets were compared to droplets comprising the native parental strain as well as to a control sample droplet, without initial exposure to UV light.

UV-C irradiation was used in the mutagenesis approach is known to severely impact on the viability of bacteria due to excessive DNA damage. In other words, if the DNA damage resulting from UV-C irradiation is too severe the viability of cells and, as a consequence, the efficacy of the direct evolution process is compromised.

In order to create a library of bacteria with a large fraction of viable mutants, the effect of UV-C irradiation dose on the survival rate of cells was investigated in greater detail.

To test this, a liquid culture of *K. sucrofermentans* was irradiated with UV-C light (wavelength 254 nm) at doses up to 100 mJ/cm$^2$. After UV exposure, the irradiated samples were incubated in the dark for 1 hours and then suspended in rich culture mediumto allow the cells to recover from the stress of UV exposure. The bacteria were incubated for 1h to 50h at 28°C, which is the conventional culture temperature of *K. sucrofermentans.* The incubation in the dark served to inhibit the onset of DNA repair mechanisms, which counteracts the UV-induced mutagenesis. To quantify the cell survival rate, bacterial culture samples exposed to different light doses were directly frozen for subsequent colony forming unit (CFU) counting analysis.

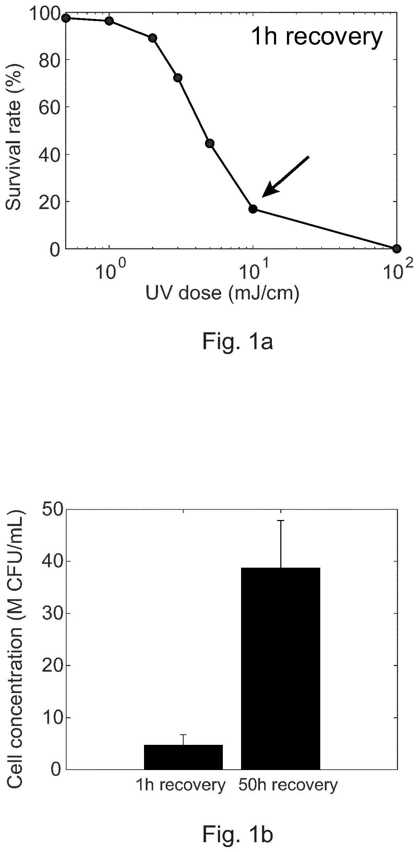

The survival rate of cell suspensions exposed to different UV-C doses compared to a non-exposed cell suspension is shown in Figure 1a. The survival rate was calculated relative to the control sample, which underwent the same steps but was not irradiated. The cell counting analysis revealed that the survival rate of the irradiated bacteria decreased from 98% to 17% upon an increase in UV dose from 0.5 mJ/cm$^2$ to 10 mJ/cm$^2$. Exposure to the highest dose of 100 mJ/cm$^2$ led to severe DNA damage and complete cell death. Based on these results, a dose of 10 mJ/cm$^2$, which is indicated by an arrow in Figure 1a, was found to be most appropriate to ensure a high fraction of mutants in the population while keeping a reasonable percentage of bacteria alive. The survival rate under these radiation conditions was about 17%.

The viability of cells exposed to 10 mJ/cm$^2$ was confirmed by growing the bacteria for 50 hours in rich culture medium In this time interval, the concentration of mutant bacteria was found to increase by more than 8-fold between 1h of incubation and 50h of incubation. As shown in Figure 1b, the determined cell concentration was 4.7 M CFU/ml, when recovering for 1h, and 38.7 M CFU/ml, when the cells were allowed to recover for 50h. Error bars represent the standard deviations.

The relatively concentrated cell suspension obtained after a 50h recovery period was the used as feedstock for the microfluidic encapsulation process.

Mutant bacteria were encapsulated in droplets using a microfluidic device via a step emulsification approach. The diameter of the droplets was about 49 μm. By operating at a high throughput rate in the order of thousands of droplets/second and reaching a droplet polydispersity index (PDI) of only 10$^{-3}$, the microfluidic platform provided a large library of mutants which was ideal for directed evolution experiments.

In the emulsification process, droplets of the recovered, diluted bacteria suspension were emulsified in a fluorocarbon oil with the help of a biocompatible surfactant, in this case a fluoro-surfactant. The number of cells encapsulated in a single microfluidic droplet is known to follow Poisson statistics and to depend on the concentration of bacteria in the feedstock suspension.

The concentration of the bacterial suspension was adjusted (λ = 0.1 CFU/droplet) to obtain a droplet population with about 90.5% of empty droplets, about 9.0% of droplets containing a single encapsulated cell, and about 0.5% of droplets with more than 1 cell.

An important task in droplet-based directed evolution processes is the appropriate design of an assay for the fast, on-chip quantification of the performance of encapsulated mutants. Given the ease and availability of fluorescent-based detection tools, we used the Fluorescent Brightener 28 to quantify the production of cellulose by the encapsulated bacteria. This commercially available dye is known to selectively bind to the (1-4)β bonds present in cellulose. To detect cellulose formation inside the droplets during incubation, we added the dye directly into the bacterial suspension used in the encapsulation process. The cellulose-forming capabilities of the encapsulated bacteria was measured after incubation of the monodisperse droplets at 28°C for up to 4 days. Measurements were performed in a confocal microscope using a 405 nm laser and a 432-460 nm detector.

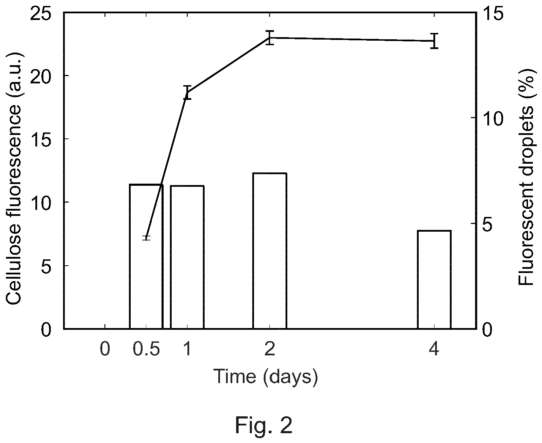

The results of these measurements are shown in Figure 2. It was observed that the majority of the cellulose was produced within the first day of droplet incubation, with a maximum fluorescence reached after two days of incubation (left axis). Error bars correspond to the standard error of the mean. About 7% of droplets contained a cellulose-producing bacteria, which is close to the 9.5% value expected from Poisson statistics for a cell concentration λ of 0.1 CFU/droplet (right axis).

Confocal microscopy images taken during incubation show that 6 hours were already sufficient for the bacteria to start producing cellulose inside the droplets, as shown in Figures 3a to 3d. The results indicate that their confinement in droplets does not impair the ability of the microorganism to synthesize cellulose. At this early stage, the cellulose was observed to form primarily around the encapsulated bacteria, as seen in Figure 3b.

When extending the incubation period to 24 hours, the bacteria-derived cellulose is no longer limited to the bacteria but occupies most of the volume of the droplets, as seen in Figure 3c. Figures 3a to 3f show fluorescence distribution data obtained by image analysis, wherein Figures 3a, 3c and 3e display representative confocal microscopy images of single-cell-laden droplets over the incubation time indicated in the respective figure. The droplets were exposed to an excitation wavelength of 405 nm, and an emission range of 432 nm to 460 nm was detected. The scale bar represents 100 μm. These Figures clearly evidence the emergence of a population of cellulose-containing droplets after 6 and 24 hours of incubation. These results validate the fluorescence-based approach to quantify the cellulose-producing capabilities of the mutant bacteria encapsulated in droplets. Figures 3b, 3d, and 3f show the corresponding histograms of cellulose fluorescence per droplet as quantified by image analysis with a sample size of about 1'200 droplets.

Due to the high emulsification rate of 2000 droplets/second, the microfluidic platform enabled the generation of a library of 1.2 million droplets in 10 minutes. This corresponds to approximately 100'000 single-cell encapsulated mutants. A fraction of this large pool of mutants (430'000 droplets, ~40'000 mutants) was further analyzed in a microfluidic droplet sorter to select for possible cellulose overproducers after 24 hours of droplet incubation off-chip. Selection was performed by fluorescence-activated droplet sorting (FADS) in a high-speed customized microfluidic device (800 Hz). The fluorescence emitted by the produced cellulose was measured as the droplet moved along the main microfluidic channel. A photomultiplier tube (PMT) was used to quantify the droplet fluorescence at 488 nm using a 405 nm laser for excitation. The PMT voltage was utilized as a proxy for the fluorescence emitted by the droplet. Experimental results underlined the the importance of a recovery time of 50 hours after UV-C exposure to obtain a strong fluorescent signal after 1 day of incubation.

A bifurcation at the end of the main channel was used to separate cellulose overproducers from the pool of mutants. The few droplets with fluorescence above a pre-defined threshold are di-electrophoretically pulled into an oulet channel of sorted bacteria, while the vast majority was discarded in a waste outlet. The PMT voltage threshold was arbitrarily set to 2.75 V to separate the 1.25% most fluorescent variants from the pool.

Sorted droplets containing evolved bacteria were collected and spread on solid media to enable the growth of individual mutants into colonies. Five colonies of evolved bacteria (Ev1 to Ev5) were chosen for further analysis. The evolved bacteria were compared to native and control strains by measuring their cellulose-forming capabilities inside droplets or in bulk pellicles. For the analysis in droplets, the bacteria were re-encapsulated ($\lambda$ ~0.1 CFU/droplet), incubated for 24h and screened using the same microfluidic platform used for the initial evolution experiment. This time no sorting was performed. Instead, the analysis of the fluorescence intensity distribution obtained for the evolved, native and control bacteria was performed directly.

The evolved mutants clearly outperformed the native and control bacteria in terms of cellulose production inside droplets. Fluorescence intensity histograms obtained for these three variants show a Gaussian-like distribution that is strongly skewed towards high fluorescence value, as observed in Figure 4a, showing histograms of cellulose fluorescence signal in droplets containing native, control and evolved (Ev5) bacterial cells, with $\lambda$ ~0.1 CFU/droplet and measurements for approximately 15'000 droplet events.

To quantify the performance of the distinct bacteria, we fitted the distributions with a Gaussian mixture function and compared the values of peak fluorescence intensity. The evolved bacteria displayed a mean cellulose fluorescence that is 52% higher than that measured for the native strain, whereas the control strain displayed a 36% increase compared to the native strain. This enhanced cellulose-producing capability is also evidenced by the stronger maximum fluorescence signal measured for droplets loaded with the evolved bacteria.

Importantly, the over-production of cellulose by the evolved bacteria is not limited to droplets but can also be translated into the bulk manufacturing of thicker cellulose pellicles. To illustrate this, the evolved, native and control bacterial cells from single colonies were cultivated in a liquid culture medium which allowed for the growth of a BC pellicle at the air-water interface in static conditions. A long incubation time of 12 days was chosen to ensure cells reached the stationary growth phase and to maximize cellulose production. This also minimized the possible effect of slight differences in the initial inoculation amounts. Pellicles were then washed and dried to quantify the amount of celllulose produced by the different variants. Freeze-drying was performed to obtain macroscopic samples, whereas thermogravimetric analysis and weight measurements with a laboratory scale were carried out to quantify the amount of cellulose formed by the different strains.

Visual inspection of the freeze-dried films clearly showed the production of thicker pellicles by the evolved bacteria compared to the native species, as the images taken of the washed and freeze-dried pellicles as Figure 4b demonstrate. Both scale measurements and thermogravimetric analysis of air-dried pellicles heated up to 650°C revealed that 4 of the 5 evolved strains chosen for analysis produced 54wt% - 70wt% more cellulose compared to the native bacteria, as depicted in Figure 4c, which shows weight measurements of washed and air-dried bacterial cellulose pellicles grown for 12 days from single colonies.

The long fibers obtained from these strains show the typical interwoven microstructure of bacterial cellulose, as shown in Figures 5a to d. The images shown in these Figures are scanning electron microscopy images of freeze-dried bacterial cellulose pellicles produced by the native parental strain (Figures 5a, 5b) and Ev5 strain (Figure 5c, 5d) after 12 days of incubation at 28°C in static conditions. Figures 5a and 5c represent the bottom fibers of the pellicle. Figures 5b and 5d show the cross-section of the pellicle. The long fibers produced by both strains are of similar appearance.

Interestingly, the control strain also showed an increase in cellulose production by 19% relative to the native species, suggesting that the stresses imposed to the bacteria during the treatments for mutagenesis, such as lack of nutrients, darkness, and/or recovery time, promote cellulose formation. These results also indicate that the mutants selected for fast cellulose production in droplets also led to bulk fims with the highest total cellulose mass after incubation for 12 days.

To evaluate the stability of the enhanced phenotype, passage experiments in which bacterial cellulose pellicles were grown from previous pellicles over multiple generation, were performed. The results demonstrate that the higher production of cellulose by the selected evolved strain (Ev5) was stable even after 5 passages.

*K. sucrofermentans* strain Ev5 was deposited with the Culture Collection of Switzerland (CCOS) under the accession number CCOS 2107.

In addition to this stability experiment, an additional directed evolution cycle of the Ev5 strain to explore the possibility of further phenotype improvement was performed. To this end, the evolved strain was mutated, encapsulated, incubated, and sorted with two different thresholds: (i) the same condition as in the first round (2.75 V) and (ii) more stringent selection pressure (3.60 V). The selected strains of this second round of directed evolution did not show a further increase in cellulose production, but maintained a similar production as in the first evolution round.

In addition to producing cellulose in the form of thick bulk pellicles or in the form of pellets or granules, the ability of the evolved bacteria to overproduce cellulose also opens new opportunities for the manufacturing of more complex engineered living materials.

To further explore this possibility, a 3D printable gel that can be loaded with the evolved cellulose-producing bacteria was designed. Networking-forming silica particles and viscosity-modifiers hyaluronic acid and $\kappa$-carrageenan were used to tune the rheological properties of the gel according to a known protocol (Schaffner M et al, 2017, "3D Printing of Bacteria into Functional Complex Materials", Sci Adv. 3, eaao6804). Oscillatory rheology experiments confirmed that gels with or without cells display elastic response at low shear stresses and become a viscous fluid above a well-defined yield stress of 200 Pa. These properties fullfil the rheological requirements for 3D printing via the extrusion-based direct ink writing (DIW) technique.

Cell-laden gels were successfully printed into a three-dimensional object with complex geometry at the centimeter scale, an example of which is shown in Figure 6a. In addition to the rheology modifiers, the ink also contained the nutrients required for the proliferation and growth of the embedded bacteria. This allowed for the in-situ production of cellulose fibers within the printed gel during an incubation period of 1 day.

The evolved bacteria may therefore be used to generate cellulose-based macroscopic objects using state-of-the-art manufacturing technologies, such as casting, molding and 3D printing.

To gain insights into the effect of the bacterial strain on the formation of cellulose within the gel, we printed discs containing the cellulose-binding dye and loaded with the evolved or native strains, and imaged them in a confocal microscope. The fluorescence of the cellulose-binding dye was taken as a proxy for the local concentration of cellulose produced by the bacteria. To restrict oxygen supply to the edges of the sample, the 12-mm printed discs remained sandwiched between a Petri dish and a cover slip during incubation and imaging.

Confocal images of the printed discs revealed that the evolved bacteria produce cellulose predominantly along the edge of the sample next to the air-water interface (Figure 6c). This contrasts with the less localized and more uniform distribution of cellulose in discs containing the native strain (Figure 6b). Radially integrated fluorescence measurements of the samples further provided clear evidence of the distinct cellulose patterns created by the two bacterial strains. This example illustrates how the architecture of engineered living materials can be tuned at different length scales by combining the top-down manufacturing capabilities of 3D printing with the bottom-up self-assembly processes controlled by microorganisms.

The biological machinery that controls the self-assembly of cellulose fibers in *K. sucrofermentans* can be affected by mutations in the bacterial genome or by metabolic changes resulting from environmental stresses the bacteria are exposed to during the mutagenesis process.

To establish the origin of the enhanced cellulose production in the evolved strains, the genome of the evolved bacteria was compared with that of the native strain. For this, a high-quality reference genome was obtained, using both shotgun and long-read methods to sequence the native strain. The assembled genome contained a single 2.95 Mbp genome and 4 plasmids, as schematically depicted in Figure 7. The genomes of the evolved and control strains were sequenced with the Illumina platform and aligned to the reference genome. The breseq algorithm known in the art was used to predict mutations from annotated sequences.

The genomic analysis revealed no mutation in the control strain but a consistent and unique mutation in the 4 evolved strains found to be overproducers of cellulose (Ev2, Ev3, Ev4, and Ev5): a 12-base pair deletion, i.e. nucleotides 2081 to 2070, within the reading frame of the *clpA* gene, as shown in Figure 7. The reverse complement strand of the *clpA* gene is transcribed for expression. The missing nucleotides code for a sequence of four amino acids of the N-terminal domain of the ClpA protein, i.e. amino acids QRVI at position 84 to 87 in the ClpA polypeptide.

Surprisingly, this highly significant deletion was found in a gene encoding a polypeptide of the ClpAPS protease complex, and not in any of *bcs* genes or their homologs encoding the multiple protein domains forming the cellulose synthase complex.

The ClpA protein is part of the ClpAPS complex, which is responsible for the hydrolysis of misfolded and degraded proteins inside cells. The function of ClpA is to bind, unfold and lead proteins to ClpP, where the hydrolysis process occurs. Under normal conditions, the proteins to be degraded are fixed through the specific interaction between the N-terminal domain of ClpA with the adaptor protein ClpS, as previously demonstrated for the *E. coli* ClpAPS complex.

In fact, it could be established that the deleted 12 base pairs in the mutant strains code for four amino acids of the ClpS-binding domain of ClpA. In particular, the Arginine residue corresponding to R86 in *E. coli* was shown to be highly conserved across species and involved in the ClpS bonding to ClpA (Zeth K et al, (2002), Nature Structural Biology 9, 906 - 911, doi:10.1038/nsb869).

Zeth et al describe both the ClpA binding region in ClpS and the ClpS binding region in ClpA in various bacterial species and identify the conserved amino acids in said binding regions.

The authors of this study localized the ClpA binding region from amino acid 75 to 85 in the ClpS polypeptide [SEQ ID NO 11,12]. The sequence of the binding region of *E. coli* was identified (V75-V85) as VFTA<u>E</u>VA<u>E</u>T<u>K</u>V, wherein the underlined residues are highly conserved ClpA-binding residues for six different homologs investigated as part of the study, i.e. E79, E72 and K84. One of the homologs was the ClpS polypeptide of *A. tumefaciens,* which is a known cellulose producer. The ClpA binding region in ClpS of *A. tumefaciens* is : VFTY<u>E</u>VA<u>E</u>T<u>K</u>V. Other homologous sequences are provided in Figure 1e of Zhet et al and are hereby incorporated by reference. It was shown that a combination of two point mutation (E79A and K84A) abolished ClpA binding and as a result all ClpAPS-mediated activities.

In the same document, Zeth et al describe two ClpS binding regions in the N-terminal domain, i.e. residue 1 to residue 161, of the *E.coli* ClpA polypeptide [SEQ ID NO 9, 10], i.e. a first region from residue 20 to residue 30, and a second region from residue 80 to residue 90. The identified sequences in *E. coli* of the first region is HRH<u>E</u>FMTVE<u>HL</u>, and of the second region is P<u>T</u>LSF<u>QR</u>VLQR, with the highly conserved residues being underlined. Other homologous sequences are provided in Figure 2c of Zhet et al and are hereby incorporated by reference. Residue R86 residue in the ClpS binding region of ClpA was conserved across species, and it is assumed to be essential for ClpS binding.

The sequence data of the native *K. sucrofermentans* confirmed the presence of the ClpA binding region in the ClpS polypeptide [SEQ ID NO 7, 8] from residue V136 to residue V146, having the sequence VFTY<u>E</u>VAE<u>T</u>KV, and the presence of the two ClpS binding regions in the ClpA polypeptide [SEQ ID NO 5, 6] from residue R20 to residue L30, having the sequence RRHEYATLEHL, and from residue P79 to residue R89 having the sequence PT<u>A</u>AFC<u>QR</u>VIQR. Residues Q84 to I87, including the highly conserved residue R85, are deleted in the cellulose overproducing *K. sucrofermentans* mutant strains EV2, Ev3, Ev4 and Ev5 of this invention.

Without wishing to be bound by any particular hypothesis, it is therefore assumed that a reduction in ClpS-ClpA binding affinity or an abolishment of ClpS-ClpA binding contributes to the observed increased cellulose production of the evolved bacterial strains Ev2, Ev3, Ev4 and Ev5.

*K. sucrofermentans* strain Ev5 was named *K. sucrofermentans* JML 2321, both names designate the same strain.

Further support of this hypothesis is based on data obtained from a ⊿*clpS* knockout strain, which was generated from the native *K. sucrofermentans* strain by homologous recombination. The successful deletion of the *clpS* gene in the knockout strain was confirmed by gel electrophoresis. The ⊿*clpS* knockout strain was compared with the native *K. sucrofermentans* strain as well as to the *K. sucrofermentans* Ev5 strain in terms of cellulose formation in bulk pellicles as well as bacterial growth in liquid culture medium.

As shown in Figure 8, the ⊿*clpS* knockout strain produced 17wt% more cellulose than the native strain. The observed increase in cellulose production by the ⊿*clpS* strain points to a so far unknown connection between ClpS-ClpA interaction and cellulose regulation. However, the knock-out mutant produced significantly less cellulose than the evolved Ev5 strain, which, in the same experiment produced 37wt% more cellulose than the native parental strain of *K. sucrofermentans*.

ClpS was previously shown to change the degrading specificity of the ClpAP protease complex. The results of this study suggest that such a reduction or a change in protein degradation specificity might favour cellulose production in *K. sucrofermentans*. This finding is in agreement with earlier research on *C. glutamicum*, which showed that a clpS deletion results in a significant increase in expression of a heterologous protein.

Interestingly, cell growth experiments revealed that the control strain, the evolved Ev5 strain, and the ⊿*clpS* strain showed accelerated growth rates compared to the native *K. sucrofermentans* parental strain (Figure 9).

The fact that the ⊿*clpS* knockout strain did not reach the 37% increase in cellulose production observed in the evolved overproducers indicates that other factors than the ClpS-ClpA interaction might also contribute to the increased cellulose biosynthesis in the evolved bacteria. Since the control strain showed an accelerated growth rate and also produced 19% more cellulose than the native parental strain it is speculated that the stresses imposed on the bacteria during the treatment steps necessary for mutagenesis to account for at least part of the non-genetic contributions to the enhanced cellulose production.

In addition, it was observed that sorted cells of the native *K. sucrofermentans* parental strain also exhibited a 17% increase in cellulose production compared to native, unsorted cells.

Without wishing to be bound by any partiular hypothesis, the results indicate that in addition to the relevant genetic mutation, , physiological stresses may also contribute to the increased cellulose production of of *K. sucrofermentans.*

The link between clp genes and the overproduction of cellulose established by this invention opens new biotechnological avenues for rendering bacterial cellulose production more efficient and attractive for industrial purposes.

Material and Methods

Cell culture for bacterial cellulose (BC) production

*Komagataeibacter sucrofermentans* JCM 9730 (ATCC 700178) is the cellulose-producing strain used in this study. Their growth media was composed of 25 g/L D-mannitol (Thermo Fisher Scientific), 5 g/L yeast extract (Sigma-Aldrich), 3 g/L peptone (Sigma-Aldrich), and optionally 15 g/L agar (Sigma-Aldrich) for solid medium. Frozen stocks of *K. sucrofermentans* (-80°C) were streaked on media plates to isolate single colonies, which were then inoculated in 5 ml growth media in 50 ml Falcon tubes (TPP). The lid was replaced with a foam plug to allow optimal oxygen availability. The cultures were incubated in static conditions at 28°C for 8-12 days to form bacterial cellulose (BC) pellicles at the air-media interface. For passage experiments (phenotype retention), a sterile loop was used to rub the BC pellicles and streaked on solid medium, from which single colonies were picked for subsequent liquid cultures.

Cell concentration

All experiments were started from frozen stocks of bacteria. For each stock, serial dilutions from $10^{-1}$ to $10^{-6}$ were prepared and plated on solid media in drops of

5 μl (n = 6). Colony-forming units (CFU) were counted at an appropriate dilution, averaged, and the stock concentration was back-calculated.

## Absorbance measurements

Absorbance measurements were taken with the Varioscan LUX (Thermo Fisher Scientific) at 600 nm, on 200 μl samples in 96-well plates (flat bottom, TPP) unless stated otherwise. Samples were always measured in triplicates, averaged, and blanks were subtracted. Blanks corresponded to media, with 2 vol% cellulase (Trichoderma reesei ATCC 26921, Sigma-Aldrich) and 35 μg/ μl chloramphenicol (Sigma-Aldrich) only when also present in the samples.

## UV-C mutagenesis

*K. sucrofermentans* from frozen stocks were inoculated in 150 ml of liquid media for 6 days at 28°C at 200 rpm. 2% cellulase was added to the culture to digest any cellulose produced. After measuring the absorbance, cells were spun down at 3275 rcf for 10 min (Z306 Hermle) and resuspended in 0.9% NaCl (VWR) to reach a theoretical absorbance of 1. 10 ml of the cell suspension was added to each Petri dish (Ø10 cm, TPP) to be exposed to different UV-C doses without the lid: 0, 0.5, 1, 2, 3, 5, 10, and 100 mJ/cm$^2$ (254 nm, UVP Crosslinker CL-3000, AnalytikJena). The plates were then left in the dark for 1h, spun down as previously, resuspended in enriched media (2X concentration) with 2% cellulase, and incubated at 28°C and 200 rpm for either 1 h or 50 h. To store the cultures at -80°C for further analysis, aliquots with 20% glycerol (Fisher BioReagents) were prepared. To quantify the cell survival, the 1 h cultures were serial diluted and plated on solid media (5 μl dots, n = 6). Colony-forming units (CFU) were counted at the 10$^{-4}$ dilution and compared to the Control which was not exposed to UV-C (0 mJ/cm$^2$). The 50 h aliquots were used for the directed evolution process, assuming more recovery time was beneficial to increase our chances of finding a strain which grows sufficiently well in our culture media.

## Fabrication of microfluidic devices

SU-8 (3000 series, MicroChem) was patterned on a Silicon wafer using standard photolithography methods. Those were used as masters for 1:10 PDMS (Sylgard™ 184, Dow Corning) soft lithography. After curing, the devices were peeled off, inlet and outlet holes were punched, and the devices were air-plasma bonded to glass slides (Fisherbrand™ Superfrost™). To hydrophobize the devices, a 2 vol% solution of 1H,1H,2H,2H-perfluorooctyltrichlorosilane (Fluorochem) in HFE-7500 (3M) was flushed through the inlet and then air dried. For the sorting devices, electrodes were created by inserting a low melting point solder (51In/32.5Bi/16.5Sn; Indium Corporation, New York, USA) at one end of the electrode channel and a wire at the other end.

## Single-cell encapsulation and incubation

*Cell loading:* The desired cell loading was evaluated with the well-known Poisson distribution representing the probability of having k cells in a droplet knowing the average number of cells per droplet volume $\lambda$:

$$p(k, \lambda) = \frac{\lambda^k e^{-\lambda}}{k!}$$

. Knowing the droplet size and frozen stock concentration, we aimed at a $\lambda$ of 0.1 CFU/droplet, corresponding to a ~9.5% droplet occupancy. This low number was chosen to minimize co-encapsulation events (<0.5%). For each encapsulation experiment, cells were thawed from frozen stocks, and their concentration was adjusted to a $\lambda$ of 0.1 CFU/droplet (~1.5M CFU/mL). The occupancy was validated by image analysis, estimating the percentage of droplets in which cellulose was produced (see Droplet image analysis section).

*Cell encapsulation:* Cells were encapsulated in ~50 um droplets using a step emulsification PDMS microfluidic device. The inner aqueous phase was composed of media, cells at low concentration ($\lambda$ = 0.1 cells/droplet), 218 uM Fluorescent Brightener 28 (FB, Sigma-Aldrich) to stain the BC, and 26 uM fluorescein (Sigma-Aldrich) to get a background signal in the droplets. The outer phase comprised 2 wt% 008-FluoroSurfactant in HFE-7500 (RAN Biotechnologies). Both phases were flown at 500 uL/h for 10 minutes, corresponding to more than a million droplets formed.

*Droplet incubation:* Droplets were incubated in hydrophobized glass vials, on top of 200 μL HFE-7500 (3M), at 28°C in static conditions for 24h before the sorting process. The vials were horizontally placed to guarantee a monolayer of droplets and equal access to oxygen.

## Droplet image analysis

Prior to screening in microfluidic devices, droplet sizes and cellulose content were analyzed using z-stacks of confocal images (30 slices, 3.58 um each). A 405 nm laser was used to excite the cellulose-specific dye (Fluorescent Brightener 28, Sigma-Aldrich), which was detected between 432-460 nm (HyD detector). An additional PMT transmission detector was active to image the droplets. Using ImageJ, [42] the FB images were summed, and one of the droplet images was chosen for edge detection. Droplet diameters were estimated using the Hough Transform plugin (UCB Vision Sciences) after thresholding the image. For fluorescence quantification in the droplets, the FB sum stack and chosen droplet images were imported to Cell Profiler 4.1.3, a user-friendly software previously reported for droplet image analysis. Briefly, droplets were detected as objects in a desirable size range and filtered out if on the edge of the image, and then the object intensity of each object was measured. Those results were exported as an Excel file, and all were analyzed and plotted with MATLAB (R2023a, Math Works). Droplets were considered occupied when their measured FB fluorescence was exceeding the maximum fluorescence detected on Day 0.

Droplet sorting

Droplets were then transferred to a second PDMS microfluidic device, in which they were spaced with oil (HFE-7500, 3M). After passing under a 405 nm laser, the fluorescently labeled cellulose intensity was estimated with a 488 nm PMT detector, which we expect to be proportional to the voltage detected. Based on the fluorescein background in the droplets, the baseline was adjusted to 0.05 V. 0.43M droplets were detected in less than 10 min, of which 505 were sorted with a threshold of 2.75 V. Those droplets were collected in a sterile Eppendorf tube. After adding media and demulsifying, the droplets were plated onto solid media. Five evolved single colonies were then picked and grown in liquid media with 2% cellulase, and aliquots with 20% glycerol (Fisher BioReagents) were frozen for further analysis (Ev1-Ev5).

Cellulose dry weight

To quantify the amount of BC produced by the evolved strains compared to the native and control (0 mJ/cm$^2$) strains, triplicates of BC pellicles were grown as previously explained. After 12 days, they were washed 3 times with 0.1 M NaOH (Fisher Scientific, UK) at 60°C in a water bath over a period of 24h and then brought back to neutral pH washing 3 times with MilliQ water (NANOpure Diamond, Barnstead). They were then dried in a 60°C oven for 48h on Teflon films (McMater-Carr, OH) to avoid sticking, and stored under vacuum until analyzed. Dried bacterial cellulose pellicles were weighed with a precision balance (UMT2 Microbalance, Mettler Toledo). Thermogravimetric analysis (Discovery TGA 5500, TA Instruments) was as well performed on the dried cellulose pellicles by increasing the temperature of 10°C/min until 650°C, with an isotherm of 15 min at 120°C to remove all the humidity contained in the samples. Weight loss was then calculated between the end of the 120°C isotherm and the end of the program at 650°C.

Genome sequencing, assembly and analysis

Genome sequencing was performed by MicrobesNG (United Kingdom). The *K. sucrofermentans* DSM 15973 reference genome was sequenced with an Illumina NovaSeq 6000 (Illumina, San Diego, USA) using a 250 bp paired end protocol, as well as with GridION (Oxford Nanopore Technologies, UK) to obtain long reads. Strains selected from the directed evolution process were sequenced with Illumina NovaSeq 6000 (Illumina, San Diego, USA) only.

Knockout generation

*K. sucrofermentans* knockout was produced by transforming cells with a pUC19 plasmid, which does not replicate inside this bacterium. Further, the pUC19 plasmid was engineered to contain 500 bp regions of homology to the genome around the *clpS* sequence, designed to remove the start codon of *clpS* and disrupt expression. The homology arms were placed on each side of a *cat* chloramphenicol resistance cassette, to create plasmid pUC19-clpS-KO through Gibson assembly and cloning in *E. coli* DH5a cells.

*K. sucrofermentans* cells were made electrocompetent by growing a culture to an absorbance of 0.8 at 600 nm, before centrifuging at 3000 g for 10 min and washing the pellets three times with 10% glycerol (Fisher BioReagents). Cells were then concentrated and electroporated with pUC19-clpS-KO. Following overnight recovery in media supplemented with 2 vol% cellulase at 28°C in shaking conditions (200 rpm), cells were plated onto 25 µg/ml chloramphenicol to select for colonies containing the *cat* cassette. Colonies were screened by colony PCR with GoTaq polymerase (M7423, Promega), using verification primers designed around the insertion site in *clpS* on the genome. PCR products were run in 1.5% agarose (Ultrapure Agarose, Thermo Fisher Scientific) with gel electrophoresis in Tris-Acetate-EDTA (TAE) buffer (Fisher Bioreagents). Sybr Safe (Thermo Fisher Scientific) DNA stain was added initially to the gel, and after electrophoresis imaging was performed using a ChemiDoc™ MP imaging system (BioRad). Further verification was performed by Sanger Sequencing PCR products with the verification primers (Microsynth, Switzerland), which in each case found the expected sequence.

3D printing

*Ink Preparation:* The 4.5% ink is composed of 1.5 wt% sodium hyaluronate (BulkSupplements), 1.5 wt% κ-carrageenan (Acros Organics), and 1.5 wt% fumed silica (WDK V15, Wacker Chemie) in the standard media used for *K. sucrofermentans* described above. The ink was prepared following the previously published protocol (Schaffner, M et al. (2017): 3D Printing of Bacteria into Functional Complex Materials, Sci. Adv. 3, eaao6804), UV-C sterilizing all powders before mixing and adding 218 uM of Fluorescent Brightener (Sigma-Aldrich) to stain the cellulose produced in the ink. For each experiment, a new batch of ink was prepared and separated in three equal amounts, in which 0.36M CFU/g of bacteria were added from frozen stocks. For controls, the same volume of sterile media was added (900 uL). The final inks were loaded into 10 ml syringes and kept at 4°C until 3D printing on the same day.

*Ink Rheology:* Rheological measurements of the inks were performed with a sandblasted parallel plate geometry (PP25-S, Anton Paar) at 25°C (MCR 302 compact rheometer, Anton Paar). The storage and loss moduli (G' and G") were analyzed with constant oscillatory measurements (1 s$^{-1}$) with shear strain amplitude increasing logarithmically from 0.01 to 100%. Flow curves were then recorded with rotational strain-controlled measurements with a shear rate ramping logarithmically from 0.01 to 100 s$^{-1}$ and then from 100 to 0.01 s$^{-1}$.

*3D printing*: All structures were 3D printed using a 10 ml syringe, a 0.84 mm needle, and a layer height of 0.7 mm. 1-layer disks of 12 mm diameter were 3D printed directly on Petri dishes. Immediately after the print was done, the disks were covered with coverslips, so oxygen was only available from the sides. Each Petri dish was then sealed with parafilm and imaged as is with a confocal microscope (TCS SP8, Leica) after a day of incubation at 28°C and 85% RH in static conditions. For each full disk, a 15-slice stack of 750 um total was imaged (excitation: 405 nm; emission: 432-460 nm) and stitched together using the Leica software.

*Image analysis:* All the image analysis was performed using ImageJ. Slices of the stacks were averaged, and the radial profile of each disk was plotted. Data was then smoothed and plotted with MATLAB (R2023a, Math Works).

Growth curves

Each tested strain was thawed, spun down at 3000 rcf for 10 min (5417R, Eppendorf), resuspended in 1 ml of media, and their absorbance was adjusted to 0.005. 2% of cellulase was added to each culture to digest the produced cellulose. 1 ml of each culture was then transferred to 24-well plates (flat bottom, TPP) in triplicates, and the well plates were covered with a breathable film (BREATHseal™, Greiner Bio-One) to avoid contamination between wells. The well plates were incubated at 28°C and 85% RH in shaking conditions (200 rpm). Every day, the breathable film was removed for the measurement, the absorbance at 600 nm of each well was measured, triplicates were averaged, and blanks composed of media and 2% cellulase were subtracted from the measurement. The breathable film was changed, and the plates were incubated until the next measurement.

Statistical analysis

The significance of the cellulose increased production was evaluated with a one-way ANOVA (MATLAB R2023a, Math Works), followed by a pairwise comparison if the results showed a statistically significant difference between the groups ($p < 0.05$). A Bonferroni correction was applied to compensate for the effects of multiple comparisons.

**Claims**

1. A genetically modified cellulose producing bacterial strain comprising

 - a reduction in expression of native gene encoding a ClpS polypeptide, or a deletion of the native gene encoding a native ClpS polypeptide,
or comprising
 - expressing or overexpressing a gene encoding a ClpA polypeptide comprising a mutation or a deletion in the ClpS-binding region of said polypeptide, and/or expressing or overexpressing a gene encoding a ClpS polypeptide comprising a mutation or deletion in the ClpA-binding region of said polypeptide, causing an alteration of binding affinity between the ClpS polypeptide and the ClpA polypeptide.

2. The genetically modified bacterial strain of claim 1, wherein the mutation or deletion on the ClpS-binding region of ClpA, and/or the mutation or deletion on the ClpA-binding region of ClpS abolishes binding of ClpS to ClpA, or causes a reduction in binding affinity between the ClpS polypeptide and the ClpA polypeptide.

3. The genetically modified bacterial strain of claim 1 or 2, comprising a mutation or a deletion of one or more of the following conserved residues in the gene sequence encoding the the ClpS-binding region of the ClpA polypeptide:

 - the conserved arginine (R) residue corresponding to the R86 residue in the homologous *Escherichia coli* ClpA polypeptide,
 - one or both of the conserved glutamic acid (E) residues corresponding to the E23 residue or the E28 residue in the homologous *Escherichia coli* ClpA polypeptide, and/or
 - the conserved threonine(T) residue corresponding to the T81 residue in the homologous *Escherichia coli* ClpA polypeptide.

4. The genetically modified bacterial strain of any of claims 1 to 3, comprising a mutation or a deletion of one or more of the following conserved residues in the gene sequence encoding the conserved residues in the ClpA-binding region of the ClpS polypeptide:

 - one or both of the conserved E residues corresponding to the E79 or to the E82 residue in the homologous *Escherichia coli* ClpS polypeptide, and/or
 - the conserved lysine (K) residue corresponding to the K84 residue in the homologous *Escherichia coli* ClpS polypeptide.

5. The genetically modified bacterial strain of any of claims 1 to 4, comprising a mutation or a deletion of the gene sequence encoding at least 12 nucleotides encoding four amino acids of the ClpS-binding region of the ClpA polypeptide, said four amino acids corresponding to positions 85 to 88 of the homologous *E. coli* ClpA polypeptide.

6. The genetically modified bacterial strain of any of claim 1 to 5, wherein the binding affinity between the expressed or overexpressed ClpA polypeptide and the expressed or overexpressed ClpS is reduced by at least 90%, or by at least 80%, or by at least 70%, or by at least 60%, or by at least 50%, or by at least 40%, or by at least 30%, or by at least 20%.

7. The genetically modified bacterial strain of any of claim 1 to 6, having reduced ClpAP protease activity.

8. The genetically modified bacterial strain of any of claim 1 to 7, being a naturally cellulose-producing bacterial strain selected from the group consisting of *Komagataeibacter* sp, for example *Komagataeibacter sucrofermentans, Gluconacetobacter* sp, for example *Gluconacetobacter xylinus, Agrobacterium* spp., for example *Agrobacterium tumefaciens, Acetobacter* spp., *Azotobacter, Rhizobium* spp., *Sarcina, Alcaligenes,* and *Pseudomonas.*

9. The genetically modified bacterial strain of any of claim 1 to 8, which is designated *Komagataeibacter sucrofermentans* JML 2321 having CCOS accession number CCOS 2107, or a *Komagataeibacter sucrofermentans* strain having a growth profile and a cellulose production profile of CCOS deposit accession number CCOS 2107.

10. The genetically modified bacterial strain of any of claim 1 to 9, which is a naturally cellulose-producing bacterial strain.

11. The genetically modified bacterial strain of any of claim 1 to 10, which is a recombinant non-naturally cellulose producing bacterial strain expressing or overexpressing a polypeptide or a polypeptide system having cellulose synthase activity, or expressing or overexpressing the genes encoding the BC synthase complex *bcsA, bcsB, bcsC and bcsD* or their homologs and, optionally, *cmcax* and *ccpAx* genes or homologs thereof.

12. The genetically modified bacterial strain of any of claim 1 to 11, producing at least 15%, or at least 20%, 30%, 40%, 50%, 60%, 70%, 80%, 90%, 100% more cellulose that its parental strain,

13. Method for producing cellulose comprising growing the genetically modified bacterial strain of any of claims 1 to 12 in a static or in an agitated culture to obtain a bacterial cellulose film or bacterial cellulose pellets.

14. Use of the modified bacterial strain of any of claims 1 to 12 in a manufacturing process, for example a casting, molding, or 3D printing process, for the production of cellulose-based objects.

Fig. 1a

Fig. 1b

Fig. 2

Fig. 3a

Fig. 3b

Fig. 3c

Fig.3d

Fig. 3e

Fig. 3f

Fig. 4a

Fig. 4b

Fig. 4c

Fig. 5a

Fig. 5b

Fig. 5c

Fig. 5d

Fig. 6a

Fig. 6b

Fig. 6c

Fig. 7

Fig. 8

Fig. 9

Europäisches
Patentamt

European
Patent Office

Office européen
des brevets

# EUROPEAN SEARCH REPORT

**Application Number**

EP 23 21 0958

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DAVID A DOUGAN ET AL: "ClpS, a Substrate Modulator of the ClpAP Machine two nucleotide binding domains (NBDs). Although bind- ing of ATP is sufficient to promote hexamer assembly", MOLECULAR CELL, vol. 9, no. 3, 1 March 2002 (2002-03-01), pages 673-683, XP055557403, * page 676, right-hand column * | 1-14 | INV. C12P19/04 |
| A | XIUXIA LIU: "Effect of Clp protease from Corynebacterium glutamicum on heterologous protein expression", PROTEIN EXPRESSION AND PURIFICATION, vol. 189, 1 January 2022 (2022-01-01), page 105928, XP093166424, SAN DIEGO, CA. ISSN: 1046-5928, DOI: 10.1016/j.pep.2021.105928 Retrieved from the Internet: URL:https://www.sciencedirect.com/science/article/pii/S104659282100111X/pdfft?md5=ca80634dd7bb1bb175c341cc83c31beb&pid=1-s2.0-S104659282100111X-main.pdf> * page 2, right-hand column - page 3, right-hand column * | 1-14 | TECHNICAL FIELDS SEARCHED (IPC) C12P |

-/--

The present search report has been drawn up for all claims

| Place of search | Date of completion of the search | Examiner |
|---|---|---|
| Munich | 24 May 2024 | Stoyanov, Borislav |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 23 21 0958

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ANNETTE H ERBSE ET AL: "Conserved residues in the N-domain of the AAA+ chaperone ClpA regulate substrate recognition and unfolding", THE FEBS JOURNAL, WILEY-BLACKWELL PUBLISHING LTD, GB, vol. 275, no. 7, 14 February 2008 (2008-02-14), pages 1400-1410, XP072370252, ISSN: 1742-464X, DOI: 10.1111/J.1742-4658.2008.06304.X * abstract * | 1-14 | |
| X | BULDUM GIZEM ET AL: "Recombinant biosynthesis of bacterial cellulose in genetically modifiedEscherichia coli", BIOPROCESS AND BIOSYSTEMS ENGINEERING, SPRINGER, DE, vol. 41, no. 2, 24 November 2017 (2017-11-24), pages 265-279, XP036392924, ISSN: 1615-7591, DOI: 10.1007/S00449-017-1864-1 [retrieved on 2017-11-24] | 1,6,7, 11-14 | |
| A | * abstract * | 2-5,8-10 | **TECHNICAL FIELDS SEARCHED (IPC)** |
| A | KORNELIUS ZETH: "Structural analysis of the adaptor protein ClpS in complex with the N-terminal domain of ClpA", NATURE STRUCTURAL BIOLOGY., vol. 9, no. 12, 11 November 2002 (2002-11-11), pages 906-911, XP093166436, NEW YORK, NY, US ISSN: 1072-8368, DOI: 10.1038/nsb869 * figure 1E * | 1-14 | |

The present search report has been drawn up for all claims

| Place of search | Date of completion of the search | Examiner |
|---|---|---|
| Munich | 24 May 2024 | Stoyanov, Borislav |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
 
& : member of the same patent family, corresponding
   document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BULDOM G et al.** *Bioprocess and Biosystems Eng*, 2018, vol. 41, 265-279
- **SCHAFFNER M et al.** 3D Printing of Bacteria into Functional Complex Materials''. *Sci Adv*, 2017, vol. 3, eaao6804
- **ZETH K et al.** *Nature Structural Biology*, 2002, vol. 9, 906-911
- **SCHAFFNER, M et al.** 3D Printing of Bacteria into Functional Complex Materials. *Sci. Adv.*, 2017, vol. 3, eaao6804